(19) Europäisches Patentamt
European Patent Office
Office européen des brevets

(11) EP 2 046 401 B1

(12) **EUROPÄISCHE PATENTSCHRIFT**

(45) Veröffentlichungstag und Bekanntmachung des Hinweises auf die Patenterteilung:
**26.01.2011 Patentblatt 2011/04**

(21) Anmeldenummer: **07787355.2**

(22) Anmeldetag: **11.07.2007**

(51) Int Cl.:
***A61L 15/22*** *(2006.01)* ***A61L 15/60*** *(2006.01)*
***C08F 2/10*** *(2006.01)* ***C08F 20/04*** *(2006.01)*
***C08J 3/24*** *(2006.01)*

(86) Internationale Anmeldenummer:
**PCT/EP2007/057081**

(87) Internationale Veröffentlichungsnummer:
**WO 2008/009598 (24.01.2008 Gazette 2008/04)**

(54) **VERFAHREN ZUR HERSTELLUNG WASSERABSORBIERENDER POLYMERPARTIKEL MIT HOHER PERMEABILITÄT DURCH POLYMERISATION VON TROPFEN EINER MONOMERLÖSUNG**

METHOD FOR PRODUCING WATER-ABSORBENT POLYMER PARTICLES WITH A HIGHER PERMEABILITY BY POLYMERISING DROPLETS OF A MONOMER SOLUTION

PROCÉDÉ PERMETTANT LA PRODUCTION DE PARTICULES POLYMÈRES ABSORBANT L'EAU PRÉSENTANT UNE PERMÉABILITÉ ÉLEVÉE PAR POLYMÉRISATION DE GOUTELLETTES D'UNE SOLUTION MONOMÈRE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **19.07.2006 EP 06117491**

(43) Veröffentlichungstag der Anmeldung:
**15.04.2009 Patentblatt 2009/16**

(73) Patentinhaber: **BASF SE**
**67056 Ludwigshafen (DE)**

(72) Erfinder:
- **STUEVEN, Uwe**
**65812 Bad Soden (DE)**
- **WEISMANTEL, Matthias**
**63637 Jossgrund (DE)**
- **HEIDE, Wilfried**
**67251 Freinsheim (DE)**
- **KRÜGER, Marco**
**68161 Mannheim (DE)**
- **SEIDL, Volker**
**68199 Mannheim (DE)**
- **BLEI, Stefan**
**68163 Mannheim (DE)**
- **LÖSCH, Dennis**
**67122 Altrip (DE)**
- **FUNK, Rüdiger**
**65527 Niedernhausen (DE)**
- **HILLEBRECHT, Annemarie**
**36100 Petersberg (DE)**

(56) Entgegenhaltungen:
**EP-A- 1 424 346**
**WO-A-2005/080479**
**DE-A1-102004 057 868**
**US-B1- 6 291 605**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 046 401 B1

Printed by Jouve, 75001 PARIS (FR)

## Beschreibung

Die vorliegende Erfindung betrifft ein Verfahren zur Herstellung wasserabsorbierender Polymerpartikel mit hoher Permeabilität durch Polymerisation von Tropfen einer Monomerlösung in einer die Tropfen umgebenden Gasphase, wobei die Monomerlösung bezogen auf das Monomer mindestens 0,5 Gew.-% eines Vernetzers enthält, die Polymerisation im Tropfen in homogener Phase stattfindet und die Polymerpartikel einem mittleren Durchmesser von mindestens 150 μm aufweisen.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Wasserabsorbierende Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Die Eigenschaften der wasserabsorbierenden Polymere können über den Vernetzungsgrad eingestellt werden. Mit steigendem Vernetzungsgrad steigt die Gelfestigkeit und sinkt die Absorptionskapazität. Dies bedeutet, dass mit steigender Absorption unter Druck (AUL) die Zentrifugenretentionskapazität (CRC) abnimmt (zu sehr hohen Vernetzungsgraden nimmt auch die Absorption unter Druck wieder ab).

Zur Verbesserung der Anwendungseigenschaften, wie beispielsweise Flüssigkeitsweiterleitung im gequollenen Gelbett (SFC) in der Windel und Absorption unter Druck (AUL), werden wasserabsorbierende Polymerpartikel im allgemeinen nachvernetzt. Dadurch steigt nur der Vernetzungsgrad der Partikeloberfläche, wodurch die Absorption unter Druck (AUL) und die Zentrifugenretentionskapazität (CRC) zumindest teilweise entkoppelt werden können. Diese Nachvernetzung kann in wässriger Gelphase durchgeführt werden. Vorzugsweise werden aber gemahlene und abgesiebte Polymerpartikel (Grundpolymer) an der Oberfläche mit einem Nachvernetzer beschichtet, thermisch nachvernetzt und getrocknet. Dazu geeignete Vernetzer sind Verbindungen, die mindestens zwei Gruppen enthalten, die mit den Carboxylatgruppen des hydrophilen Polymeren kovalente Bindungen bilden können.

Durch Sprühpolymerisation konnten die Verfahrensschritte Polymerisation und Trocknung zusammengefasst werden. Zusätzlich konnte die Partikelgröße durch geeignete Verfahrensführung in gewissen Grenzen eingestellt werden. Die Herstellung wasserabsorbierender Polymerpartikel durch Polymerisation von Tropfen einer Monomerlösung wird beispielsweise in EP-A 0 348 180, WO 96/40427, US 5,269,980, DE-A 103 14 466, DE-A 103 40 253 und DE-A 10 2004 024 437 sowie den älteren deutschen Anmeldungen mit den Aktenzeichen 10 2005 002 412.2 und 10 2006 001 596.7 beschrieben.

DE-A 10 2004 042 946, DE-A 10 2004 042 948 und DE-A 10 2004 042 955 sowie die ältere deutsche Anmeldung mit dem Aktenzeichen 10 2005 019 398.6 beschreiben die Herstellung von Verdickern durch Sprühpolymerisation.

Aufgabe der vorliegenden Erfindung war die Bereitstellung eines Verfahrens zur Herstellung wasserabsorbierender Polymerpartikel mit hoher Permeabilität, d.h. einer hohen Flüssigkeitsweiterleitung durch das gequollene Gelbett.

Gelöst wurde die Aufgabe durch ein Verfahren zur Herstellung wasserabsorbierender Polymerpartikel durch Polymerisation von Tropfen einer Monomerlösung, enthaltend

a) mindestens ein wasserlösliches ethylenisch ungesättigtes Monomer,
b) mindestens einen Vernetzer,
c) mindestens einen Initiator,
d) Wasser,

in einer die Tropfen umgebenden Gasphase, wobei die Polymerisation im Tropfen in homogener Phase stattfindet, dadurch gekennzeichnet, dass die Monomerlösung mindestens 0,5 Gew.-% des Vernetzers b) bezogen auf das Monomer a) enthält und die Polymerpartikel einem mittleren Durchmesser von mindestens 150 μm aufweisen.

Die gemäß dem erfindungsgemäßen Verfahren erhältlichen wasserabsorbierenden Polymerpartikel weisen eine Permeabilität (SFC) von typischerweise mindestens 5 x $10^{-7}$ $cm^3s/g$, vorzugsweise mindestens 15 x $10^{-7}$ $cm^3s/g$, bevorzugt mindestens 35 x $10^{-7}$ $cm^3s/g$, besonders bevorzugt mindestens 120 x $10^{-7}$ $cm^3s/g$, ganz besonders bevorzugt mindestens 200 x $10^{-7}$ $cm^3s/g$, auf. Die Permeabilität (SFC) der wasserabsorbierenden Polymerpartikel beträgt üblicherweise weniger als 500 x $10^{-7}$ $cm^3s/g$.

Die gemäß dem erfindungsgemäßen Verfahren erhältlichen wasserabsorbierenden Polymerpartikel weisen eine Zentrifugenretentionskapazität (CRC) von typischerweise mindestens 10 g/g, vorzugsweise mindestens 15 g/g, bevorzugt mindestens 20 g/g, besonders bevorzugt mindestens 25 g/g, ganz besonders bevorzugt mindestens 30 g/g, auf. Die Zentrifugenretentionskapazität (CRC) der wasserabsorbierenden Polymerpartikel beträgt üblicherweise weniger als 50 g/g.

Die Monomerlösung enthält vorzugsweise mindestens 0,6 Gew.-%, bevorzugt mindestens 0,8 Gew.-%, besonders bevorzugt mindestens 1,5 Gew.-%, ganz besonders bevorzugt mindestens 3,0 Gew.-%, Vernetzer b), jeweils bezogen auf Monomer a).

Der mittlere Durchmesser der Polymerpartikel beträgt vorzugsweise mindestens 200 µm, besonders bevorzugt von 250 bis 600 µm, ganz besonders von 300 bis 500 µm, wobei der Partikeldurchmesser durch Lichtstreuung bestimmt werden kann und den volumengemittelten mittleren Durchmesser bedeutet. 90% der Polymerpartikel weisen einen Durchmesser von vorzugsweise 100 bis 800 µm, besonders bevorzugt von 150 bis 700 µm, ganz besonders bevorzugt von 200 bis 600 µm, auf.

Der Sauerstoffgehalt der Gasphase beträgt vorzugsweise 0,001 bis 0,15 Vol.-%, besonders bevorzugt 0,002 bis 0,1 Vol.-%, ganz besonders bevorzugt 0,005 bis 0,05 Vol.-%.

Die Gasphase enthält neben Sauerstoff vorzugsweise nur inerte Gase, d.h. Gase, die unter Reaktionsbedingungen nicht in die Polymerisation eingreifen, beispielsweise Stickstoff und/oder Wasserdampf.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 50 g/100 g Wasser, und haben vorzugsweise mindestens je eine Säuregruppe.

Die Konzentration der Monomeren a) in der Monomerlösung beträgt üblicherweise 2 bis 80 Gew.-%, vorzugsweise 5 bis 70 Gew.-%, besonders bevorzugt 10 bis 60 Gew.-%.

Geeignete Monomere a) sind beispielsweise ethylenisch ungesättigte Carbonsäuren, wie Acrylsäure, Methacrylsäure, Maleinsäure, Fumarsäure und Itaconsäure. Besonders bevorzugte Monomere sind Acrylsäure und Methacrylsäure. Ganz besonders bevorzugt ist Acrylsäure.

Die bevorzugten Monomere a) haben mindestens eine Säuregruppe, wobei die Säuregruppen vorzugsweise zumindest teilweise neutralisiert sind.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Säuregruppen der Monomere a) sind üblicherweise teilweise neutralisiert, vorzugsweise zu 25 bis 85 mol-%, bevorzugt zu 50 bis 80 mol-%, besonders bevorzugt 60 bis 75 mol-%, wobei die üblichen Neutralisationsmittel verwendet werden können, vorzugsweise Alkalimetallhydroxide, Alkalimetalloxide, Alkalimetallcarbonate oder Alkalimetallhydrogencarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumcarbonat oder Natriumhydrogencarbonat sowie deren Mischungen. Üblicherweise wird die Neutralisation durch Einmischung des Neutralisationsmittels als wässrige Lösung, als Schmelze, oder bevorzugt auch als Feststoff erreicht. Beispielsweise kann Natriumhydroxid mit einem Wasseranteil deutlich unter 50 Gew.-% als wachsartige Masse mit einem Schmelzpunkt oberhalb 23°C vorliegen. In diesem Fall ist eine Dosierung als Stückgut oder Schmelze bei erhöhter Temperatur möglich.

Die Monomere a), insbesondere Acrylsäure, enthalten vorzugsweise bis zu 0,025 Gew.-% eines Hydrochinonhalbethers. Bevorzugte Hydrochinonhalbether sind Hydrochinonmonomethylether (MEHQ) und/oder Tocopherole.

Unter Tocopherol werden Verbindungen der folgenden Formel verstanden

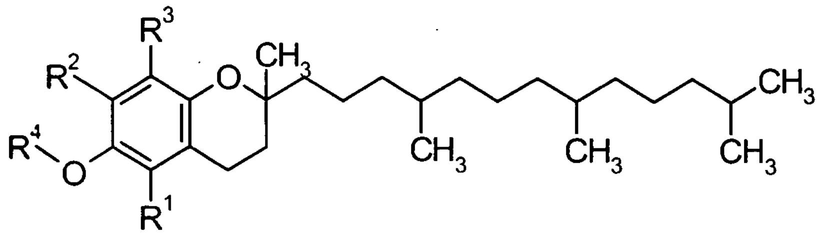

wobei $R^1$ Wasserstoff oder Methyl, $R^2$ Wasserstoff oder Methyl, $R^3$ Wasserstoff oder Methyl und $R^4$ Wasserstoff oder ein Säurerest mit 1 bis 20 Kohlenstoffatomen bedeutet.

Bevorzugte Reste für $R^4$ sind Acetyl, Ascorbyl, Succinyl, Nicotinyl und andere physiologisch verträgliche Carbonsäuren. Die Carbonsäuren können Mono-, Di- oder Tricarbonsauren sein.

Bevorzugt ist alpha-Tocopherol mit $R^1 = R^2 = R^3$ = Methyl, insbesondere racemisches alpha-Tocopherol. $R^1$ ist besonders bevorzugt Wasserstoff oder Acetyl. Insbesondere bevorzugt ist RRR-alpha-Tocopherol.

Die Monomerlösung enthält bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf Acrylsäure, wobei Acrylsäuresalze als Acrylsäure mit berücksichtigt werden. Beispielsweise kann zur Herstellung der Monomerlösung eine Acrylsäure mit einem entsprechenden Gehalt an Hydrochinonhalbether verwendet werden.

Die Vernetzer b) sind Verbindungen mit mindestens zwei radikalisch polymerisierbaren Gruppen, die in das

Polymernetzwerk radikalisch einpolymerisiert werden können. Geeignete Vernetzer b) sind beispielsweise Ethylenglykoldimethacrylat, Diethylenglykoldiacrylat, Allylmethacrylat, Trimethylolpropantriacrylat, Triallylamin, Tetraallyloxyethan, wie in EP-A 0 530 438 beschrieben, Di- und Triacrylate, wie in EP-A 0 547 847, EP-A 0 559 476, EP-A 0 632 068, WO 93/21237, WO 03/104299, WO 03/104300, WO 03/104301 und in DE-A 103 31 450 beschrieben, gemischte Acrylate, die neben Acrylatgruppen weitere ethylenisch ungesättigte Gruppen enthalten, wie in DE-A 103 314 56 und DE-A 103 55 401 beschrieben, oder Vernetzermischungen, wie beispielsweise in DE-A 195 43 368, DE-A 196 46 484, WO 90/15830 und WO 02/32962 beschrieben.

Geeignete Vernetzer b) sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen, wie Allyl(meth) acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A 0 343 427 beschrieben sind. Weiterhin geeignete Vernetzer b) sind Pentaerythritoldi- Pentaerythritoltri- und Pentaerythritoltetraallylether, Polyethylenglykoldiallylether, Ethylenglykoldiallylether, Glyzerindi- und Glyzerintriallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 300 und 1.000 aufweist.

Besonders vorteilhafte Vernetzer b) sind jedoch Di- und Triacrylate des 3- bis 15-fach ethoxylierten Glyzerins, des 3- bis 15-fach ethoxylierten Trimethylolpropans, des 3- bis 15-fach ethoxylierten Trimethylolethans, insbesondere Di- und Triacrylate des 2- bis 6-fach ethoxylierten Glyzerins oder Trimethylolpropans, des 3-fach propoxylierten Glyzerins oder Trimethylolpropans, sowie des 3-fach gemischt ethoxylierten oder propoxylierten Glyzerins oder Trimethylolpropans, des 15-fach ethoxylierten Glyzerins oder Trimethylolpropans, sowie des 40-fach ethoxylierten Glyzerins, Trimethylolethans oder Trimethylolpropans.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine wie sie beispielsweise in WO 03/104301 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5- fach ethoxylierten und/oder propoxylierten Glyzerins. Am meisten bevorzugt sind die Triacrylate des 3- bis 5-fach ethoxylierten und/oder propoxylierten Glyzerins. Diese zeichnen sich durch besonders niedrige Restgehalte (typischerweise unter 10 Gew.-ppm) im wasserabsorbierenden Polymer aus und die wässrigen Extrakte der damit hergestellten wasserabsorbierenden Polymere weisen eine fast unveränderte Oberflächenspannung (typischerweise mindestens 0,068 N/m) im Vergleich zu Wasser gleicher Temperatur auf.

Als Initiatoren c) können sämtliche unter den Polymerisationsbedingungen in Radikale zerfallende Verbindungen eingesetzt werden, beispielsweise Peroxide, Hydroperoxide, Wasserstoffperoxid, Persulfate, Azoverbindungen und die sogenannten Redoxinitiatoren. Bevorzugt ist der Einsatz von wasserlöslichen Initiatoren. In manchen Fällen ist es vorteilhaft, Mischungen verschiedener Initiatoren zu verwenden, beispielsweise Mischungen aus Wasserstoffperoxid und Natrium- oder Kaliumperoxodisulfat. Mischungen aus Wasserstoffperoxid und Natriumperoxodisulfat können in jedem beliebigen Verhältnis verwendet werden.

Besonders bevorzugte Initiatoren c) sind Azoinitiatoren, wie 2,2'-Azobis[2-(2-imidazolin-2-yl)propan]dihydrochlorid und 2,2'-Azobis[2-(5-methyl-2-imidazolin-2-yl)propan]dihydrochlorid, und Photoinitiatoren, wie 2-Hydroxy-2-methylpropiophenon und 1-[4-(2-Hydroxyethoxy)-phenyl]-2-hydroxy-2-methyl-1-propan-1-on, Redoxinitiatoren, wie Natriumpersulfat/ Hydroxymethylsulfinsäure, Ammoniumperoxodisulfat/Hydroxymethylsulfinsäure, Wasserstoffperoxid/Hydroxymethylsulfinsäure, Natriumpersulfat/Ascorbinsäure, Ammoniumperoxodisulfat/Ascorbinsäure und Wasserstoffperoxid/Ascorbinsäure, Photoinitiatoren, wie 1-[4-(2-Hydroxyethoxy)-phenyl]-2-hydroxy-2-methyl-1-propan-1-on, sowie deren Mischungen.

Die Initiatoren werden in üblichen Mengen eingesetzt, beispielsweise in Mengen von 0,001 bis 5 Gew.-%, vorzugsweise 0,01 bis 1 Gew.-%, bezogen auf die Monomeren a).

Die bevorzugten Polymerisationsinhibitoren benötigen für eine optimale Wirkung gelösten Sauerstoff. Daher kann die Monomerlösung vor der Polymerisation durch Inertisierung, d. h. Durchströmen mit einem inerten Gas, vorzugsweise Stickstoff, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, gesenkt.

Die Polymerisationsinhibitoren können auch durch Absorption, beispielsweise an Aktivkohle, entfernt werden.

Die Monomerlösung wird zur Polymerisation in der Gasphase vertropft.

Die Polymerisation in den Monomerlösungstropfen findet in homogener Phase statt. Dies bedeutet, dass die Monomerlösung homogen ist und dass die Monomerlösung auch während der Polymerisation homogen bleibt. Das Polymer darf während der Polymerisation quellen, aber nicht ausfallen und eine zweite Phase im Tropfen bilden. Ansonsten würden in jedem Tropfen mehrere Polymerkeime entstehen, die während der Trocknung Agglomerate sehr kleiner Primärpartikel bilden. Ziel des erfindungsgemäßen Verfahrens ist die Herstellung jeweils eines Primärpartikels pro Tropfen. Daher sind die Monomeren a) und die Vernetzer b) so auszuwählen, dass das entstehende Polymer in der wässrigen Phase des Tropfens quellbar ist.

Daher wird das erfindungsgemäße Verfahren vorzugsweise in Abwesenheit hydrophober inerter Lösungsmittel durchgeführt. Hydrophobe, inerte Lösungsmittel sind praktisch alle mit Wasser nicht mischbaren Flüssigkeiten, die nicht in die Polymerisation eingreifen, d.h. keine polymerisierbaren Gruppen enthalten. Mit Wasser nicht mischbar bedeutet, dass die Löslichkeit der hydrophoben Lösungsmittel in Wasser weniger als 5 g/100 g, vorzugsweise weniger als 1 g/ 100 g, besonders bevorzugt weniger als 0,5 g/100 g, beträgt.

Bei der Vertropfung wird eine Monomerlösung unter Ausbildung von Tropfen in die Gasphase dosiert. Die Vertropfung der Monomerlösung kann beispielsweise mittels einer Vertropferplatte durchgeführt werden.

Eine Vertropferplatte ist eine Platte mit mindestens einer Bohrung, wobei die Flüssigkeit von oben durch die Bohrung tritt. Die Vertropferplatte bzw. die Flüssigkeit kann in Schwingungen versetzt werden, wodurch an der Unterseite der Vertropferplatte je Bohrung eine idealerweise monodisperse Tropfenkette erzeugt wird.

Die Anzahl und die Größe der Bohrungen werden gemäß der gewünschten Kapazität und Tropfengröße ausgewählt. Der Tropfendurchmesser beträgt dabei üblicherweise das 1,9fache des Durchmessers der Bohrung. Wichtig ist hierbei, dass die zu vertropfende Flüssigkeit nicht zu schnell durch die Bohrung tritt bzw. der Druckverlust über die Bohrung nicht zu groß ist. Ansonsten wird die Flüssigkeit nicht vertropft, sondern der Flüssigkeitsstrahl wird infolge der hohen kinetischen Energie zerrissen (versprüht). Der Vertropfer wird im Strömungsbereich des laminaren Strahlzerfalls betrieben, d. h. die Reynoldszahl bezogen auf den Durchsatz pro Bohrung und den Bohrungsdurchmesser ist vorzugsweise kleiner als 2.000, bevorzugt kleiner 1.000, besonders bevorzugt kleiner 500, ganz besonders bevorzugt kleiner 100. Der Druckverlust über die Bohrung beträgt vorzugsweise weniger als 2,5 bar, besonders bevorzugt weniger als 1,5 bar, ganz besonders bevorzugt weniger als 1 bar.

Die Vertropferplatte weist üblicherweise mindestens eine, vorzugsweise mindestens 10, besonders bevorzugt mindestens 50, und üblicherweise bis zu 10.000, vorzugsweise bis zu 5.000, besonders bevorzugt bis zu 1.000, Bohrungen auf, wobei die Bohrungen üblicherweise gleichmäßig über die Vertropferplatte verteilt sind, vorzugsweise in der sogenannten Dreiecksteilung, d.h. jeweils drei Bohrungen bilden die Ecken eines gleichseitigen Dreiecks.

Der Durchmesser der Bohrungen wird an die gewünschte Tropfengröße angepasst.

Es kann vorteilhaft sein die Vertropferplatte auf eine Trägerplatte aufzulegen, wobei die Trägerplatte ebenfalls Bohrungen aufweist. Dabei weisen die Bohrungen der Trägerplatte einen größeren Durchmesser auf als die Bohrungen der Vertropferplatte und sind so angeordnet, dass sich unter jeder Bohrung der Vertropferplatte eine mit ihr konzentrische Bohrung der Trägerplatte befindet. Diese Anordnung ermöglicht einen schnellen Wechsel der Vertropferplatte, beispielsweise um Tropfen einer anderen Größe zu erzeugen.

Die Vertropfung kann aber auch mittels pneumatischer Ziehdüsen, Rotation, Zerschneiden eines Strahls oder schnell ansteuerbarer Mikroventildüsen durchgeführt werden.

In einer pneumatischen Ziehdüse wird ein Flüssigkeitsstrahl zusammen mit einem Gasstrom durch eine Blende beschleunigt. Über die Gasmenge kann der Durchmesser des Flüssigkeitsstrahls und damit der Tropfendurchmesser beeinflusst werden.

Bei der Vertropfung durch Rotation tritt die Flüssigkeit durch die Öffnungen einer rotierenden Scheibe. Durch die auf die Flüssigkeit wirkende Fliehkraft werden Tropfen definierter Größe abgerissen. Die Rotationsvertropfung wird beispielsweise in DE-A 4308842 und US 6338438 beschrieben.

Der austretende Flüssigkeitsstrahl kann aber auch mittels eines rotierenden Messers in definierte Segmente zerschnitten werden. Jedes Segment bildet anschließend einen Tropfen.

Bei Verwendung von Mikroventildüsen werden direkt Tropfen mit definiertem Flüssigkeitsvolumen erzeugt.

Bevorzugt strömt die Gasphase als Trägergas durch den Reaktionsraum. Dabei kann das Trägergas im Gleichstrom oder im Gegenstrom zu den frei fallenden Tropfen der Monomerlösung durch den Reaktionsraum geführt werden, bevorzugt im Gleichstrom. Vorzugsweise wird das Trägergas nach einem Durchgang zumindest teilweise, bevorzugt zu mindestens 50%, besonders bevorzugt zu mindestens 75%, als Kreisgas in den Reaktionsraum zurückgeführt. Üblicherweise wird eine Teilmenge des Trägergases nach jedem Durchgang ausgeschleust, vorzugsweise bis zu 10%, besonders bevorzugt bis zu 3%, ganz besonders bevorzugt bis zu 1 %.

Die Polymerisation wird vorzugsweise in einer laminaren Gasströmung durchgeführt. Eine laminare Gasströmung ist eine Gasströmung, bei der sich die einzelnen Schichten der Strömung nicht vermischen, sondern parallel bewegen. Ein Maß für die Strömungsverhältnisse ist die Reynolds-Zahl (Re). Unterhalb einer kritischen Reynolds-Zahl ($Re_{krit}$) von 2300 ist die Gasströmung laminar. Die Reynolds-Zahl der laminaren Gasströmung beträgt vorzugsweise weniger als 2000, besonders bevorzugt weniger als 1500, ganz besonders bevorzugt weniger als 1000. Der untere Grenzfall der laminaren Inertgasströmung ist eine ruhende Inertgasatmosphäre (Re = 0), d. h., es wird nicht kontinuierlich Inertgas eingespeist.

Die Gasgeschwindigkeit wird vorzugsweise so eingestellt, dass die Strömung im Reaktor gerichtet ist, beispielsweise liegen keine der allgemeinen Strömungsrichtung entgegengesetzte Konvektionswirbel vor, und beträgt beispielsweise 0,1 bis 2 m/s, vorzugsweise 0,5 bis 1,8 m/s, bevorzugt 1 bis 1,5 m/s.

Das Trägergas wird zweckmäßigerweise vor dem Reaktor auf die Reaktionstemperatur vorgewärmt.

Die Reaktionstemperatur beträgt bei der thermisch induzierten Polymerisation vorzugsweise 70 bis 250°C,

besonders bevorzugt 100 bis 220°C, ganz besonders bevorzugt 120 bis 200°C.

Die Reaktion kann im Überdruck oder im Unterdruck durchgeführt werden, ein Unterdruck von bis zu 100 mbar gegenüber dem Umgebungsdruck ist bevorzugt.

Das Reaktionsabgas, d.h. das den Reaktionsraum verlassende Trägergas, kann beispielsweise in einem Wärmeaustauscher abgekühlt werden. Dabei kondensieren Wasser und nicht umgesetztes Monomer a). Danach kann das Reaktionsabgas zumindest teilweise wieder aufgewärmt und als Kreisgas in den Reaktor zurückgeführt werden. Ein Teil des Reaktionsabgases kann ausgeschleust und durch frisches Trägergas ersetzt werden, wobei im Reaktionsabgas enthaltenes Wasser und nicht umgesetzte Monomere a) abgetrennt und rückgeführt werden können.

Besonders bevorzugt ist ein Wärmeverbund, dass heißt, ein Teil der Abwärme beim Abkühlen des Abgases wird zum Aufwärmen des Kreisgases verwendet.

Die Reaktoren können begleitbeheizt werden. Die Begleitheizung wird dabei so eingestellt, dass die Wandtemperatur mindestens 5°C oberhalb der Reaktorinnentemperatur liegt und die Kondensation an den Reaktorwänden zuverlässig vermieden wird.

Das Reaktionsprodukt kann dem Reaktor in üblicher Weise entnommen werden, beispielsweise am Boden über eine Förderschnecke, und gegebenenfalls bis zur gewünschten Restfeuchte und zum gewünschten Restmonomerengehalt getrocknet werden.

Selbstverständlich können die Polymerpartikel anschließend zur weiteren Verbesserung der Eigenschaften nachvernetzt werden.

Geeignete Nachvernetzer sind Verbindungen, die mindestens zwei Gruppen enthalten, die mit den Carboxylatgruppen des Hydrogels kovalente Bindungen bilden können. Geeignete Verbindungen sind beispielsweise Alkoxysiliylverbindungen, Polyaziridine, Polyamine, Polyamidoamine, Di- oder Polyglycidylverbindungen, wie in EP-A 0 083 022, EP-A 0 543 303 und EP-A 0 937 736 beschrieben, di- oder polyfunktionelle Alkohole, wie in DE-C 33 14 019, DE-C 35 23 617 und EP-A 0 450 922 beschrieben, oder β-Hydroxyalkylamide, wie in DE-A 102 04 938 und US 6,239,230 beschrieben.

Des weiteren sind in DE-A 40 20 780 zyklische Karbonate, in DE-A 198 07 502 2-Oxazolidon und dessen Derivate, wie 2-Hydroxyethyl-2-oxazolidon, in DE-A 198 07 992 Bis- und Poly-2-oxazolidinone, in DE-A 198 54 573 2-Oxotetrahydro-1,3-oxazin und dessen Derivate, in DE-A 198 54 574 N-Acyl-2-Oxazolidone, in DE-A 102 04 937 zyklische Harnstoffe, in DE-A 103 34 584 bizyklische Amidacetale, in EP-A 1 199 327 Oxetane und zyklische Harnstoffe und in WO 03/031482 Morpholin-2,3-dion und dessen Derivate als geeignete Nachvernetzer beschrieben.

Das erfindungsgemäße Verfahren ermöglicht die Herstellung wasserabsorbierender Polymerpartikel mit einer hohen Permeabilität (SFC) und einer hohen Zentrifugenretentionskapazität (CRC). Für diese Eigenschaftskombination war bislang ein zusätzlicher Nachvernetzungsschritt zwingend erforderlich.

Die erfindungsgemäßen wasserabsorbierenden Polymerpartikel weisen einen Gehalt an hydrophobem Lösungsmittel von typischerweise weniger als 0,005 Gew.-%, vorzugsweise weniger als 0,002 Gew.-%, besonders bevorzugt weniger als 0,001 Gew.-%, ganz besonders bevorzugt weniger als 0,0005 Gew.-%, auf. Der Gehalt an hydrophoben Lösungsmittel kann gaschromatographisch bestimmt werden, beispielsweise mittels der Head-Space-Technik.

Polymerpartikel, die durch umgekehrte Suspensionspolymerisation erhalten wurden, enthalten üblicherweise noch ca. 0,01 Gew.-% des als Reaktionsmediums verwendeten hydrophoben Lösungsmittels.

Die erfindungsgemäßen wasserabsorbierenden Polymerpartikel weisen einen Tensidgehalt von typischerweise weniger als 1 Gew.-%, vorzugsweise weniger als 0,5 Gew.-%, besonders bevorzugt weniger als 0,1 Gew.-%, ganz besonders bevorzugt weniger als 0,05 Gew.-%, auf.

Polymerpartikel, die durch umgekehrte Suspensionspolymerisation erhalten wurden, enthalten üblicherweise noch mindestens 1 Gew.-% des zur Stabilisierung der Suspension verwendeten Tensids.

Die erfindungsgemäßen wasserabsorbierenden Polymerpartikel sind annähernd rund, d.h. die Polymerpartikel weisen eine mittlere Sphärizität von typischerweise mindestens 0.84, vorzugsweise mindestens 0,86, besonders bevorzugt mindestens 0,88, ganz besonders bevorzugt mindestens 0,9, auf. Die Sphärizität (SPHT) ist definiert als

$$SPHT = \frac{4\pi A}{U^2},$$

wobei A die Querschnittsfläche und U der Querschnittsumfang der Polymerpartikel ist. Die mittlere Sphärizität ist die volumengemittelte Sphärizität.

Die mittlere Sphärizität kann beispielsweise mit dem Bildanalysesystem Camsizer® (Retsch Technolgy GmbH; DE) bestimmt werden:

Zur Messung wird das Produkt über einen Trichter aufgegeben und mit einer Dosierrinne zum Fallschacht gefördert. Während die Partikel an einer Leuchtwand vorbeifallen werden sie wahlweise von einer Kamera erfasst. Die

aufgenommenen Bilder werden von der Software entsprechend der ausgewählten Parameter ausgewertet.

Zur Charakterisierung der Rundheit wird die im Programm mit Sphärizität gekennzeichnete Messgröße herangezogen. Angegeben sind die mittleren, mit dem Volumen gewichteten Sphärizitäten, wobei das Volumen der Partikel über den Äquivalentdurchmesser xc.., ermittelt werden. Zur Bestimmung des Äquivalentdurchmessers $xc_{min}$ wird der jeweils längste Sehnendurchmesser für insgesamt 32 unterschiedliche Raumrichtungen gemessen. Der Äquivalentdurchmesser $xc_{min}$ ist der kürzeste dieser 32 Sehnendurchmesser. Der Äquivalentdurchmesser $xc_{min}$ entspricht der Maschenweite eines Siebes, das das Partikel gerade noch passieren kann. Zur Erfassung der Partikel wird die sogenannte CCD-Zoom Kamera (CAM-Z) eingesetzt. Zur Steuerung der Dosierrinne wird ein Flächenbelegungsanteil von 0,5% vorgegeben.

Polymerpartikel mit relativ niedriger Sphärizität werden durch umgekehrte Suspensionspolymerisation erhalten, wenn die Polymerpartikel während oder nach der Polymerisation agglomeriert werden.

Die durch übliche Lösungspolymerisation (Gelpolymerisation) hergestellten wasserabsorbierenden Polymerpartikel werden nach der Trocknung gemahlen und klassiert wobei unregelmäßige Polymerpartikel erhalten werden. Die mittlere Sphärizität dieser Polymerpartikel beträgt zwischen ca. 0,72 und ca. 0,78.

Die vorliegende Erfindung betrifft ferner die Verwendung der oben genannten vernetzten wasserabsorbierenden Polymerpartikeln in Hygieneartikeln. Beispielsweise kann der Hygieneartikel wie folgt aufgebaut sein:

(A) eine obere flüssigkeitsdurchlässige Abdeckung
(B) eine untere flüssigkeitsundurchlässige Schicht
(C) einen zwischen (A) und (B) befindlichen Kern, enthaltend 10 bis 100 Gew.-% der erfindungsgemäßen wasserabsorbierenden Polymerpartikel
0 bis 90 Gew.-% hydrophiles Fasermaterial
bevorzugt 30 bis 100 Gew.-% der erfindungsgemäßen wasserabsorbierenden Polymerpartikel, 0 bis 70 Gew.-% hydrophiles Fasermaterial besonders bevorzugt 50 bis 100 Gew.-% der erfindungsgemäßen wasserabsorbierenden Polymerpartikel 0 bis 50 Gew.-% hydrophiles Fasermaterial
insbesondere bevorzugt 70 bis 100 Gew.-% der erfindungsgemäßen wasserabsorbierenden Polymerpartikel, 0 bis 30 Gew.-% hydrophiles Fasermaterial
am meisten bevorzugt 90 bis 100 Gew.-% der erfindungsgemäßen wasserabsorbierenden Polymerpartikel, 0 bis 10 Gew.-% hydrophiles Fasermaterial
(D) gegebenenfalls eine sich unmittelbar oberhalb und unterhalb des Kerns (C) sich befindende Tissueschicht und
(E) gegebenenfalls eine zwischen (A) und (C) sich befindende Aufnahmeschicht.

Unter Hygieneartikel sind dabei beispielsweise Inkontinenzeinlagen und Inkontinenzhosen für Erwachsene oder Windeln für Babys zu verstehen.

Bei der flüssigkeitsdurchlässigen Abdeckung (A) handelt es sich um die Schicht, die direkten Hautkontakt hat. Das Material hierfür besteht hierbei aus üblichen synthetischen oder halbsynthetischen Fasern oder Filme von Polyester, Polyolefine, Rayon oder natürlichen Fasern wie Baumwolle. Bei nichtgewebten Materialien sind die Fasern in der Regel durch Bindemittel wie Polyacrylate zu verbinden. Bevorzugte Materialien sind Polyester, Rayon und deren Blends, Polyethylen und Polypropylen. Beispiele für flüssigkeitsdurchlässige Schichten sind beschrieben in WO 99/57355, EP-A 1 023 883.

Die flüssigkeitsundurchlässige Schicht (B) besteht in der Regel aus einer Folie aus Polyethylen oder Polypropylen.

Der Kern (C) enthält neben den erfindungsgemäßen wasserabsorbierenden Polymerpartikeln hydrophiles Fasermaterial. Unter hydrophil ist zu verstehen, dass sich wässrige Flüssigkeiten schnell über die Faser verteilen. Für gewöhnlich ist das Fasermaterial Cellulose, modifizierte Cellulose, Rayon, Polyester wie Polyethylenterephthalat. Besonders bevorzugt werden Cellulosefasern wie Zellstoff. Die Fasern haben in der Regel einen Durchmesser von 1 bis 200 μm, bevorzugt 10 bis 100 μm. Darüberhinaus haben die Fasern eine Mindestlänge von 1 mm.

Der Aufbau und die Form von Windeln ist allgemein bekannt und beispielsweise in der WO 95/26209 Seite 66, Zeile 34 bis Seite 69, Zeile 11, DE-A 196 04 601, EP-A 0 316 518 und EP-A 0 202 127 beschrieben. Allgemein werden Windeln und andere Hygieneartikel auch in WO 00/65084, insbesondere auf Seiten 6 bis 15, WO 00/65348, insbesondere auf Seiten 4 bis 17, WO 00/35502, insbesondere Seiten 3 bis 9, DE-A 197 37 434 und WO 98/08439 beschrieben. Hygieneartikel für die Damenhygiene werden in folgenden Literaturstellen beschrieben. Die erfindungsgemäßen wasserabsorbierenden Polymerpartikel können dort eingesetzt werden. Literaturstellen Damenhygiene: WO 95/24173: Absorption Article for Controlling Odour, WO 91/11977: Body Fluid Odour Control, EP-A 0 389 023: Absorbent Sanitary Articles, WO 94/25077: Odour Control Material, WO 97/01317: Absorbent Hygienic Article, WO 99/18905, EP-A 0 834 297, US 5,762,644, US 5,895,381, WO 98/57609, WO 00/65083, WO 00/69485, WO 00/69484, WO 00/69481, US 6,123,693, EP-A 1 104 666, WO 01/24755, WO 01/00115, EP-A 0 105 373, WO 01/41692, EP-A 1 074 233. Tampons werden in folgenden Schriften beschrieben: WO 98/48753, WO 98/41179, WO 97/09022, WO 98/46182, WO 98/46181,

WO 01/43679, WO 01/43680, WO 00/61052, EP-A 1 108 408, WO 01/33962, DE-A 100 20 662, WO 01/01910, WO 01/01908, WO 01/01909, WO 01/01906, WO 01/01905, WO 01/24729. Inkontinenzartikel werden in folgenden Schriften beschrieben: Disposable Absorbent Article for Incontinent Individuals: EP-A 0 311 344 Beschreibung Seiten 3 bis 9, Disposable Absorbent Article: EP-A 0 850 623, Absorbent Article: WO 95/26207, Absorbent Article: EP-A 0 894 502, Dry Laid Fibrous Structure: EP-A 0 850 616, WO 98/22063, WO 97/49365, EP-A 0 903 134, EP-A 0 887 060, EP-A 0 887 059, EP-A 0 887 058, EP-A 0 887 057, EP-A 0 887 056, EP-A 0 931 530, WO 99/25284, WO 98/48753. Damenhygiene- und Inkontinenzartikel wird in folgenden Schriften beschrieben: Catamenial Device: WO 93/22998 Beschreibung Seiten 26 bis 33, Absorbent Members for Body Fluids: WO 95/26209 Beschreibung Seiten 36 bis 69, Disposable Absorbent Article: WO 98/20916 Beschreibung Seiten 13 bis 24, Improved Composite Absorbent Structures: EP-A 0 306 262 Beschreibung Seiten 3 bis 14, Body Waste Absorbent Article: WO 99/45973.

Zusätzlich zu den oben beschriebenen erfindungsgemäßen wasserabsorbierenden Polymerpartikeln liegen in der absorbierenden Zusammensetzung gemäß der vorliegenden Erfindung Kompositionen vor, welche die erfindungsgemäßen wasserabsorbierenden Polymerpartikel enthalten oder an denen sie fixiert sind. Jede Komposition ist geeignet, die die erfindungsgemäßen wasserabsorbierenden Polymerpartikel aufnehmen kann und die darüber hinaus in die Absorptionsschicht integriert werden kann. Eine Vielzahl derartiger Zusammensetzungen ist bereits bekannt. Eine Komposition zum Einbau der erfindungsgemäßen wasserabsorbierenden Polymerpartikel kann beispielsweise eine Fasermatrix sein, die aus einem Cellulosefasergemisch (airlaid web, wet laid web) oder aus synthetischen Polymerfasern (meltblown web, spunbonded web), oder aber aus einem Misch-Faserwerk aus Cellulosefasern und synthetischen Fasern besteht. Mögliche Fasermaterialien werden im sich anschließenden Kapitel detailliert beschrieben. Der Prozeß eines airlaid web ist beispielsweise geschildert in der Patentanmeldung WO 98/28478. Des weiteren können offenporige Schäume oder ähnliches zum Einbau wasserabsorbierender Polymerpartikel dienen.

Alternativ kann eine derartige Komposition durch Fusion zweier Einzelschichten entstehen, wobei eine oder besser eine Vielzahl an Kammern gebildet werden, die die erfindungsgemäßen wasserabsorbierenden Polymerpartikel enthalten. Ein derartiges Kammersystem ist detailliert geschildert in der Patentanmeldung EP-A 0 615 736 Seite 7, Zeilen 26 ff.

In diesem Fall sollte mindestens eine der beiden Schichten wasserdurchlässig sein. Die zweite Schicht kann entweder wasserdurchlässig oder wasserundurchlässig sein. Als Schichtenmaterial können Tissues oder sonstiges Gewebe, geschlossene oder offenporige Schäume, perforierte Filme, Elastomere oder Gewebe aus Fasermaterial zum Einsatz gelangen. Wenn die absorbierende Zusammensetzung aus einer Komposition von Schichten besteht, sollte das Schichtenmaterial eine Porenstruktur aufweisen, deren Porenabmessungen klein genug sind, um die erfindungsgemäßen wasserabsorbierenden Polymerpartikel zurückzuhalten. Obige Beispiele zur Komposition der absorbierenden Zusammensetzung schließen auch Laminate aus mindestens zwei Schichten mit ein, zwischen die die erfindungsgemäßen wasserabsorbierenden Polymerpartikel eingebaut und fixiert werden.

Generell besteht die Möglichkeit, Hydrogelpartikel innerhalb des Absorbent Cores zur Verbesserung der sogenannten Dry- und Wet-Integrity zu fixieren. Unter Dry- und Wet-Integrity versteht man die Fähigkeit, wasserabsorbierende Polymerpartikel derart in die absorbierende Zusammensetzung einzubauen, dass sie äußeren Krafteinwirkungen sowohl im nassen als auch im trockenen Zustand standhalten und es nicht zu Verschiebungen oder Austritt von hochquellfähigem Polymer kommt. Unter Krafteinwirkungen sind vor allem mechanische Belastungen zu verstehen, wie sie im Bewegungsablauf beim Tragen des Hygieneartikels auftreten, oder aber die Gewichtsbelastung, unter der der Hygieneartikel vor allem im Inkontinenzfall steht. Zur Fixierung gibt es eine Vielzahl an Möglichkeiten, die dem Fachmann bekannt sind. Beispiele wie die Fixierung durch Wärmebehandlung, Zusatz von Adhäsiven, Thermoplasten, Bindermaterialien sind verzeichnet in der Patentanmeldung WO 95/26209, Seite 37, Zeile 36 bis Seite 41, Zeile 14. Methoden zur Erhöhung der Wet Strength finden sich auch in der Patentanmeldung WO 00/36216.

Des weiteren kann die absorbierende Zusammensetzung aus einem Trägermaterial, wie z. B. einem Polymerfilm bestehen, auf dem die wasserabsorbierenden Polymerpartikel fixiert werden. Die Fixierung kann sowohl ein- als auch beidseitig vorgenommen werden. Das Trägermaterial kann wasserdurchlässig oder wasserundurchlässig sein.

In obige Kompositionen der absorbierenden Zusammensetzung werden die erfindungsgemäßen wasserabsorbierenden Polymerpartikel mit einem Gewichtsanteil von 10 bis 100 Gew.-%, bevorzugt 30 bis 100 Gew.-%, besonders bevorzugt 50 bis 100 Gew.-%, insbesondere bevorzugt 70 bis 100 Gew.-%, und am meisten bevorzugt 90 bis 100 Gew.-%, basierend auf dem Gesamtgewicht der Komposition und der wasserabsorbierenden Polymerpartikel eingebaut.

Dem Aufbau der vorliegenden erfindungsgemäßen absorbierenden Zusammensetzung liegen vielfältige Fasermaterialien zugrunde, die als Fasernetzwerk oder Matrices zum Einsatz gelangen. Mit eingeschlossen von der vorliegenden Erfindung sind sowohl Fasern natürlichen Ursprungs (modifiziert oder unmodifiziert), als auch Synthesefasern.

Einen detaillierten Überblick über Beispiele von Fasern, die in der vorliegenden Erfindung eingesetzt werden können, gibt die Patentanmeldung WO 95/26209, Seite 28, Zeile 9 bis Seite 36, Zeile 8.

Beispiele für Cellulosefasern schließen jene ein, die üblicherweise bei Absorptionsprodukten verwendet werden, wie Flauschzellstoff und Zellstoff vom Baumwolltyp. Die Materialien (Nadel- oder Laubhölzer), Herstellungsverfahren, wie chemischer Zellstoff, halbchemischer Zellstoff, chemothermischer mechanischer Zellstoff (CTMP) und Bleichverfahren sind nicht besonders eingeschränkt. So finden beispielsweise natürliche Cellulosefasern wie Baumwolle, Flachs, Seide, Wolle, Jute, Ethylcellulose und Celluloseacetat Anwendung.

Geeignete synthetische Fasern werden hergestellt aus Polyvinylchlorid, Polyvinylflourid, Polytetrafluorethylen, Polyvinylidenchlorid, Polyacrylverbindungen wie ORLON®, Polyvinylacetat, Polyethylvinylacetat, löslicher oder unlöslicher Polyvinylalkohol. Beispiele für synthetische Fasern schließen thermoplastische Polyolefinfasern, wie Polyethylenfasern (PULPEX®), Polypropylenfasern und Polyethylen-Polypropylen-Zweikomponentenfasern, Polyesterfasern, wie Polyethylenterephthalatfasern (DAC-RON oder KODEL®), Copolyester, Polyvinylacetat, Polyethylvinylacetat, Polyvinylchlorid, Polyvinylidenchlorid, Polyacryle, Polyamide, Copolyamide, Polystyrol und Copolymere der vorstehend genannten Polymere, sowie Zweikomponentenfasern aus Polyethylenterephthalat-Polyethylen-Isophthalat-Copolymer, Polyethylvinylacetat/Polypropylen, Polyethylen/Polyester, Polypropylen/Polyester, Copolyester/Polyester, Polyamidfasern (Nylon), Polyurethanfasern, Polystyrolfasern und Polyacrylnitrilfasern ein. Bevorzugt sind Polyolefinfasern, Polyesterfasern und deren Zweikomponentenfasern. Weiterhin bevorzugt sind in der Wärme haftende Zweikomponentenfasern aus Polyolefin vom Hülle-Kern-Typ und Seite-an-Seite-Typ wegen ihrer ausgezeichneten Formbeständigkeit nach der Flüssigkeitsabsorption.

Die genannten synthetischen Fasern werden bevorzugt in Kombination mit thermoplastischen Fasern eingesetzt. Bei der Hitzebehandlung migrieren letztere teilweise in die Matrix des vorhandenen Fasermaterials und stellen so beim Abkühlen Verbindungsstellen und erneute Versteifungselemente dar. Zusätzlich bedeutet der Zusatz thermoplastischer Fasern eine Erweiterung der vorliegenden Porenabmessungen nach erfolgter Hitzebehandlung. Auf diese Weise ist es möglich, durch kontinuierliches Zudosieren von thermoplastischen Fasern während der Bildung der Absorptionsschicht den Anteil thermoplastischer Fasern zum Deckblatt hin kontinuierlich zu steigern, wodurch ein ebenso kontinuierlicher Anstieg der Porengrößen resultiert. Thermoplastische Fasern können aus einer Vielzahl thermoplastischer Polymere gebildet werden, die einen Schmelzpunkt von weniger als 190°C, bevorzugt von 75 bis 175°C aufweisen. Bei diesen Temperaturen ist noch keine Schädigung der Cellulosefasern zu erwarten.

Längen und Durchmesser der vorstehend beschriebenen Synthesefasern sind nicht besonders eingeschränkt und im allgemeinen kann jede beliebige Faser mit einer Länge von 1 bis 200 mm und einem Durchmesser von 0,1 bis 100 Denier (Gramm pro 9.000 Meter) bevorzugt verwendet werden. Bevorzugte thermoplastische Fasern weisen eine Länge von 3 bis 50 mm, besonders bevorzugte eine Länge von 6 bis 12 mm auf. Der bevorzugte Durchmesser der thermoplastischen Faser liegt zwischen 1,4 und 10 Decitex, besonders bevorzugt zwischen 1,7 und 3,3 Decitex (Gramm pro 10.000 Meter). Die Form ist nicht besonders eingeschränkt und Beispiele schließen gewebeartige, schmale zylinderartige, geschnitten-/spaltgarnartige, stapelfaserartige und endlosfaserartige ein.

Die Fasern in der erfindungsgemäßen absorbierenden Zusammensetzung können hydrophil, hydrophob oder eine Kombination aus beiden sein. Gemäß der Definition von Robert F. Gould in der Publikation "Kontaktwinkel, Benetzbarkeit und Adhäsion", American Chemical Society (1964) wird eine Faser als hydrophil bezeichnet, wenn der Kontaktwinkel zwischen der Flüssigkeit und der Faser (bzw. ihrer Oberfläche) kleiner als 90° ist, oder wenn die Flüssigkeit zum spontanen Spreiten auf derselben Oberfläche tendiert. Beide Vorgänge sind in aller Regel coexistent. Umgekehrt wird eine Faser als hydrophob bezeichnet, wenn ein Kontaktwinkel von größer als 90° ausgebildet wird und kein Spreiten beobachtet wird.

Bevorzugt wird hydrophiles Fasermaterial eingesetzt. Besonders bevorzugt gelangt Fasermaterial zum Einsatz, das zur Körperseite hin schwach hydrophil und in der Region um die wasserabsorbierenden Polymerpartikel am stärksten hydrophil ist. Im Herstellungsprozeß wird durch den Einsatz von Schichten unterschiedlicher Hydrophilie ein Gradient erzeugt, der die auftreffende Flüssigkeit zum Hydrogel kanalisiert, wo letztendlich die Absorption erfolgt.

Geeignete hydrophile Fasern für den Einsatz in der erfindungsgemäßen absorbierenden Zusammensetzung sind beispielsweise Cellulosefasern, modifizierte Cellulosefasern, Rayon, Polyesterfasern wie beispielsweise Polyethylenterephthalat (DACRON®), und hydrophiles Nylon (HYDROFIL®). Geeignete hydrophile Fasern können auch erhalten werden durch Hydrophilierung hydrophober Fasern, wie beispielsweise die Behandlung thermoplastischer Fasern, erhalten aus Polyolefinen (beispielsweise Polyethylen oder Polypropylen, Polyamide, Polystyrole, Polyurethane usw.) mit Tensiden oder Silica. Aus Kostengründen und aus Gründen der Verfügbarkeit werden jedoch Cellulosefasern bevorzugt.

Die erfindungsgemäßen wasserabsorbierenden Polymerpartikel werden in das beschriebene Fasermaterial eingebettet. Dies kann auf vielfältige Weise geschehen, indem man beispielsweise mit dem Hydrogelmaterial und den Fasern zusammen eine Absorptionsschicht in Form einer Matrix aufbaut, oder durch Einlagerung wasserabsorbierender Polymerpartikel in Schichten aus Fasergemisch, wo sie letztendlich fixiert werden, sei es durch Haftmittel oder Laminierung der Schichten.

Die flüssigkeitsaufnehmende und -verteilende Fasermatrix kann dabei aus synthetischer Faser oder Cellulosefaser oder einem Gemisch aus synthetischer Faser und Cellulosefaser bestehen, wobei das Mischungsverhältnis von (100 bis 0) synthetische Faser: (0 bis 100) Cellulosefaser variieren kann. Die eingesetzten Cellulosefasern können zur Erhöhung der Formbeständigkeit des Hygieneartikels zusätzlich chemisch versteift sein.

Die chemische Versteifung von Cellulosefasern kann auf unterschiedlichen Wegen erreicht werden. Zum einen kann eine Faserversteifung erreicht werden durch Zusatz geeigneter Überzüge / Coatings zum Fasermaterial. Derartige

Zusätze schließen beispielsweise Polyamid-Epichlorhydrin-Überzüge (Kymene®557 H, Hercules, Inc. Wilmington Delaware, USA), Polyacrylamid- Überzüge (beschrieben in US-3,556,932 oder als Handelsprodukt der Marke Parez® 631 NC, American Cyanamid Co., Stamford, CT, USA), Melamin-Formaldehyd-Überzüge und Polyethylenimin-Überzüge mit ein.

Die chemische Versteifung von Cellulosefasern kann auch durch chemische Reaktion erfolgen. So kann beispielsweise die Zugabe von geeigneten Vernetzersubstanzen eine Vernetzung bewirken, die innerhalb der Faser stattfindet. Geeignete Vernetzersubstanzen sind typische Substanzen, die zur Vernetzung von Monomeren eingesetzt werden. Mit eingeschlossen, jedoch nicht limitiert darauf, sind $C_2$-$C_8$ Dialdehyde, $C_2$-$C_8$ Monoaldehyde mit saurer Funktionalität, und insbesondere $C_2$-$C_9$ Polycarbonsäuren. Spezifische Substanzen aus dieser Reihe sind beispielsweise Glutaraldehyd, Glyoxal, Glyoxylsäure, Formaldehyd und Citronensäure. Diese Substanzen reagieren mit mindestens zwei Hydroxyl-Gruppen innerhalb einer einzelnen Cellulosekette oder zwischen zwei benachbarten Celluloseketten innerhalb einer einzelnen Cellulosefaser. Durch die Vernetzung erfolgt eine Versteifung der Fasern, die durch diese Behandlung eine größere Formbeständigkeit verliehen bekommen. Zusätzlich zu ihrem hydrophilen Charakter weisen diese Fasern einheitliche Kombinationen aus Versteifung und Elastizität auf. Diese physikalische Eigenschaft ermöglicht es, die kapillare Struktur auch bei gleichzeitigem Kontakt mit Flüssigkeit und Kompressionskräften beizubehalten und ein vorzeitiges Kollabieren zu verhindern.

Chemisch vernetzte Cellulosefaseren sind bekannt und in WO 91/11162, US 3,224,926, US 3,440,135, US 3,932,209, US 4,035,147, US 4,822,453, US 4,888,093, US 4,898,642 und US 5,137,537 beschrieben. Die chemische Vernetzung bewirkt eine Versteifung des Fasermaterials, was sich letztendlich in einer verbesserten Formbeständigkeit des gesamten Hygieneartikels widerspiegelt. Die einzelnen Schichten werden durch dem Fachmann bekannte Methoden, wie beispielsweise Verschmelzen durch Wärmebehandlung, Zugabe von Schmelzklebern, Latexbindern usw. miteinander verbunden.

Beispiele für Verfahren, mit denen man eine absorbierende Zusammensetzung erhält, die beispielsweise aus einem Trägermaterial bestehen, an den ein- oder beidseitig wasserabsorbierende Polymerpartikel fixiert sind, sind bekannt und von der Erfindung mit eingeschlossen, jedoch nicht limitiert darauf.

Beispiele für Verfahren, mit denen man eine absorbierende Zusammensetzung erhält, die beispielsweise aus in ein Fasermaterial-Gemisch aus synthetischen Fasern (a) und Cellulosefasern (b) eingebetteten wasserabsorbierende Polymerpartikel (c) besteht, wobei das Mischungsverhältnis von (100 bis 0) synthetische Faser: (0 bis 100) Cellulosefaser variieren kann, schließen (1) ein Verfahren, bei dem (a), (b) und (c) gleichzeitig gemischt werden, (2) ein Verfahren, bei dem ein Gemisch aus (a) und (b) in (c) eingemischt wird, (3) ein Verfahren, bei dem ein Gemisch aus (b) und (c) mit (a) gemischt wird, (4) ein Verfahren, bei dem ein Gemisch aus (a) und (c) in (b) eingemischt wird, (5) ein Verfahren, bei dem (b) und (c) gemischt werden und (a) kontinuierlich zudosiert wird, (6) ein Verfahren, bei dem (a) und (c) gemischt werden und (b) kontinuierlich zudosiert wird, und (7) ein Verfahren, bei dem (b) und (c) getrennt in (a) eingemischt werden, ein. Von diesen Beispielen sind die Verfahren (1) und (5) bevorzugt. Die Vorrichtung, die in diesem Verfahren verwendet wird, ist nicht besonders eingeschränkt und es kann eine übliche, dem Fachmann bekannte Vorrichtung verwendet werden.

Die entsprechend erzeugte absorbierende Zusammensetzung kann optional einer Hitzebehandlung unterworfen werden, so dass eine Absorptionsschicht mit hervorragender Formbeständigkeit im feuchten Zustand resultiert. Das Verfahren zur Hitzebehandlung ist nicht besonders eingeschränkt. Beispiele schließen Hitzebehandlung durch Zufuhr heißer Luft oder Infrarotbestrahlung mit ein. Die Temperatur bei der Hitzebehandlung liegt im Bereich 60°C bis 230°C, bevorzugt zwischen 100°C und 200°C, besonders bevorzugt zwischen 100°C und 180°C.

Die Dauer der Hitzebehandlung hängt ab von der Art der synthetischen Faser, deren Menge und der Herstellungsgeschwindigkeit des Hygieneartikels. Generell beträgt die Dauer der Hitzebehandlung zwischen 0,5 Sekunden bis 3 Minuten, bevorzugt 1 Sekunde bis 1 Minute.

Die absorbierende Zusammensetzung wird im allgemeinen beispielsweise mit einer für Flüssigkeit durchlässigen Deckschicht und einer für Flüssigkeit undurchlässigen Unterschicht versehen. Weiterhin werden Beinabschlüsse und Klebebänder angebracht und so der Hygieneartikel fertiggestellt. Die Materialien und Arten der durchlässigen Deckschicht und undurchlässigen Unterschicht, sowie der Beinabschlüsse und Klebebänder sind dem Fachmann bekannt und nicht besonders eingeschränkt. Beispiele hierfür finden sich in der WO 95/26209.

Die wasserabsorbierenden Polymerpartikel werden mittels der nachfolgend beschriebenen Testmethoden geprüft.

Methoden:

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymere werden vor der Messung gut durchmischt. Flüssigkeitsweiterleitung (SFC Saline Flow Conductivity)

Die Flüssigkeitsweiterleitung einer gequollenen Gelschicht unter Druckbelastung von 0,3 psi (2070 Pa) wird,

wie in EP-A 0 640 330 beschrieben, als Gel-Layer-Permeability einer gequollenen Gelschicht aus superabsorbierendem Polymer bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP-A 0 640 330. Der Durchfluss wird automatisch erfasst.

Die Flüssigkeitsweiterleitung (SFC) wird wie folgt berechnet:

$$SFC\ [cm^3s/g] = (Fg(t=0)xL0)/(dxAxWP),$$

wobei Fg(t=0) der Durchfluss an Nacl-Lösung in g/s ist, der anhand einer linearen Regressionsanalyse der Daten Fg(t) der Durchflussbestimmungen durch Extrapolation gegen t=0 erhalten wird, L0 die Dicke der Gelschicht in cm, d die Dichte der NaCl-Lösung in g/$cm^3$, A die Fläche der Gelschicht in $cm^2$ und WP der hydrostatische Druck über der Gelschicht in dyn/$cm^2$ darstellt.

Zentrifugenretentionskapazität (CRC Centrifuge Retention Capacity)

Die Zentrifugenretentionskapazität der wasserabsorbierenden Polymerpartikel wird gemäß der von der EDANA (European Disposables and Nonwovens Association) empfohlenen Testmethode Nr. 441.2-02 "Centrifuge retention capacity" bestimmt.

Die angegebenen Werte für die Zentrifugenretentionskapazität beziehen sich auf die wasserfreien wasserabsorbierenden Polymerpartikel, d.h. die gemessenen Werte wurden entsprechend dem Wassergehalt der wasserabsorbierenden Polymerpartikel vor der Messung korrigiert. Der Wassergehalt der wasserabsorbierende Polymerpartikel wird gemäß der von der EDANA (European Disposables and Nonwovens Association) empfohlenen Testmethode Nr. 430.2-02 "Moisture content" bestimmt.

Die EDANA-Testmethoden sind beispielsweise erhältlich bei der European Disposables and Nonwovens Association, Avenue Eugène Plasky 157, B-1030 Brüssel, Belgien.

Beispiele:

14,6 kg Natriumacrylat (37,5 gew.-%ige Lösung in Wasser) und 1,4 kg Acrylsäure wurden mit 11,2 bis 336 g 15-fach ethoxiliertem Trimethylolpropantriacrylat als Vernetzer gemischt. Die Lösung wurde in einen erwärmten, mit Stickstoffatmosphäre gefüllten Vertropfungsturm vertropft (180°C, 12m Höhe, 2m Breite, Gasgeschwindigkeit 0,1 m/s im Gleichstrom). Die Dosiergeschwindigkeit betrug 16 kg/h. Die Vertropferplatte wies 37 Bohrungen à 170 μm auf. Der Durchmesser der Vertropferplatte betrug 65 mm. Der Initiator wurde kurz vor dem Vertropfer über einen statischen Mischer mit der Monomerlösung gemischt. Als Initiator wurde eine 15 gew.-%ige Lösung von 2,2'-Azobis[2-(2-imidazolin-2-yl)propan]dihydrochlorid in Wasser verwendet. Die Dosiergeschwindigkeit der Initiatorlösung betrug 0,224 kg/h. Die Gasaustrittstemperatur aus dem Vertropfungsturm betrug 130°C. Der mittlere Teilchendurchmesser der erhaltenen Polymerpartikel betrug 270 μm.

Anschließend wurden die erhaltenen wasserabsorbierenden Polymerpartikel analysiert. Die Ergebnisse sind in Tabelle 1 zusammengefasst:

Tab. 1: Einfluss der Vernetzerkonzentration

| Beispiel | Vernetzermenge | Vernetzergehalt*) | CRC [g/g] | SFC [$10^{-7}$ $cm^3s/g$] |
|---|---|---|---|---|
| 1**) | 11,2 g | 0,2 Gew.-% | 60,7 | 0 |
| 2**) | 16,8 g | 0,3 Gew.-% | 51,7 | 0 |
| 3**) | 22,4 g | 0,4 Gew.-% | 41,6 | 1 |
| 4 | 33,6 g | 0,6 Gew.-% | 36,8 | 8 |
| 5 | 44,7 g | 0,8 Gew.-% | 32,9 | 18 |
| 6 | 83,9 g | 1,5 Gew.-% | 29,1 | 41 |
| 7 | 168 g | 3,0 Gew.-% | 25,1 | 149 |

(fortgesetzt)

| Beispiel | Vernetzermenge | Vernetzergehalt*) | CRC [g/g] | SFC [$10^{-7}$ $cm^3s/g$] |
|---|---|---|---|---|
| 8 | 336 g | 6,0 Gew.-% | 22,6 | 267 |
| *) bezogen auf Acrylsäure<br>**) Vergleichsbeispiele | | | | |

## Patentansprüche

**1.** Verfahren zur Herstellung wasserabsorbierender Polymerpartikel durch Polymerisation von Tropfen einer Monomerlösung, enthaltend

a) mindestens ein ethylenisch ungesättigtes Monomer,
b) mindestens einen Vemetzer,
c) mindestens einen Initiator,
d) Wasser,

in einer die Tropfen umgebenden Gasphase, wobei die Polymerisation im Tropfen in homogener Phase stattfindet, **dadurch gekennzeichnet, dass** die Monomerlösung mindestens 0,5 Gew.-% des Vernetzers b) bezogen auf das Monomer a) enthält und die Polymerpartikel einen mittleren Durchmesser von mindestens 150 µm aufweisen.

**2.** Verfahren gemäß Anspruch 1, **dadurch gekennzeichnet, dass** das Monomer a) mindestens eine Säuregruppe aufweist.

**3.** Verfahren gemäß Anspruch 2, **dadurch gekennzeichnet, dass** die Säuregruppen des Monomeren a) zumindest teilweise neutralisiert sind.

**4.** Verfahren gemäß einem der Ansprüche 1 bis 3, **dadurch gekennzeichnet, dass** Monomer a) zu mindestens 50 mol-% Acrylsäure ist.

**5.** Verfahren gemäß einem der Ansprüche 1 bis 4, **dadurch gekennzeichnet, dass** die Polymerpartikel einen mittleren Durchmesser von mindestens 200 µm aufweisen.

**6.** Verfahren gemäß einem der Ansprüche 1 bis 5, **dadurch gekennzeichnet, dass** mindestens 90 Gew.-% der Polymerpartikel einen Durchmesser von 100 bis 800 µm aufweisen.

**7.** Verfahren gemäß einem der Ansprüche 1 bis 6, **dadurch gekennzeichnet, dass** ein Trägergas durch den Reaktionsraum strömt.

**8.** Verfahren gemäß Anspruch 7, **dadurch gekennzeichnet, dass** das den Reaktionsraum verlassende Trägergas nach einem Durchgang zumindest teilweise rückgeführt wird.

**9.** Verfahren gemäß einem der Ansprüche 1 bis 8, **dadurch gekennzeichnet, dass** der Sauerstoffgehalt des Trägergases von 0,001 bis 0,15 Vol.-% beträgt.

**10.** Verfahren gemäß einem der Ansprüche 1 bis 9, **dadurch gekennzeichnet, dass** die erhaltenen Polymerpartikel in mindestens einem weiteren Verfahrensschritt getrocknet und/oder nachvernetzt werden.

**11.** Wasserabsorbierende Polymerpartikel, wobei die Polymerpartikel eine mittlere Sphärizität von mindestens 0,84, einen Gehalt an hydrophobem Lösungsmittel von weniger als 0,005 Gew.-% und eine Permeabilität von mindestens 5 x $10^{-7}$ $cm^3s/g$ aufweisen.

**12.** Polymerpartikel gemäß Anspruch 11, **dadurch gekennzeichnet, dass** die Polymerpartikel eine Zentrifugenretentionskapazität von mindestens 10 g/g aufweisen.

**13.** Polymerpartikel gemäß Anspruch 11 oder 12, **dadurch gekennzeichnet, dass** die Polymerpartikel einen mittleren

Durchmesser von mindestens 200 μm aufweisen.

**14.** Polymerpartikel gemäß einem der Ansprüche 11 bis 13, **dadurch gekennzeichnet, dass** mindestens 90 Gew.-% der Polymerpartikel einen Durchmesser von 100 bis 800 μm aufweisen.

**15.** Polymerpartikel gemäß einem der Ansprüche 11 bis 14, **dadurch gekennzeichnet, dass** die Polymerpartikel zumindest teilweise aus polymerisierten Säuregruppen tragenden, Monomeren a) bestehen.

**16.** Polymerpartikel gemäß Anspruch 15, **dadurch gekennzeichnet, dass** die Säuregruppen der polymerisierten Monomeren a) zumindest teilweise neutralisiert sind.

**17.** Polymerpartikel gemäß Anspruch 15 oder 16, **dadurch gekennzeichnet, dass** das Monomer a) zu mindestens 50 mol% Acrylsäure ist.

**18.** Polymerpartikel gemäß einem der Ansprüche 11 bis 17, **dadurch gekennzeichnet, dass** die Polymerpartikel zu mindestens 0,5 Gew.-% aus einpolymerisiertem Vernetzter b) bestehen.

**19.** Verwendung der Polymerpartikel gemäß einem der Ansprüche 11 bis 18 zur Herstellung von Hygieneartikeln.

**20.** Hygieneartikel, enthaltend Polymerpartikel gemäß einem der Ansprüche 11 bis 18.

## Claims

**1.** A process for preparing water-absorbing polymer beads by polymerizing droplets of a monomer solution comprising

a) at least one ethylenically unsaturated monomer,
b) at least one crosslinker,
c) at least one initiator,
d) water,

in a gas phase surrounding the droplets, the polymerization in the droplet taking place in homogeneous phase, wherein the monomer solution comprises at least 0.5% by weight of the crosslinker b), based on the monomer a), and the polymer beads have a mean diameter of at least 150 μm.

**2.** The process according to claim 1, wherein the monomer a) has at least one acid group.

**3.** The process according to claim 2, wherein the acid groups of the monomer a) have been at least partly neutralized.

**4.** The process according to any of claims 1 to 3, wherein monomer a) is acrylic acid to an extent of at least 50 mol%.

**5.** The process according to any of claims 1 to 4, wherein the polymer beads have a mean diameter of at least 200 μm.

**6.** The process according to any of claims 1 to 5, wherein at least 90% by weight of the polymer beads have a diameter of from 100 to 800 μm.

**7.** The process according to any of claims 1 to 6, wherein a carrier gas flows through the reaction chamber.

**8.** The process according to claim 7, wherein the carrier gas leaving the reaction chamber is recycled at least partly after one pass.

**9.** The process according to any of claims 1 to 8, wherein the oxygen content of the carrier gas is from 0.001 to 0.15% by volume.

**10.** The process according to any of claims 1 to 9, wherein the resulting polymer beads are dried and/or postcrosslinked in at least one further process step.

**11.** Water-absorbing polymer beads which have a mean sphericity of at least 0.84, a content of hydrophobic solvent of

less than 0.005% by weight and a permeability of at least $5 \times 10^{-7}$ $cm^3s/g$.

**12.** Polymer beads according to claim 11, which have a centrifuge retention capacity of at least 10 g/g.

**13.** Polymer beads according to claim 11 or 12, which have a mean diameter of at least 200 μm.

**14.** Polymer beads according to any of claims 11 to 13, wherein at least 90% by weight of the polymer beads have a diameter of from 100 to 800 μm.

**15.** Polymer beads according to any of claims 11 to 14, which consist at least partly of polymerized acid-bearing monomers a).

**16.** Polymer beads according to claim 15, wherein the acid groups of the polymerized monomers a) have been at least partly neutralized.

**17.** Polymer beads according to claim 15 or 16, wherein monomer a) is acrylic acid to an extent of at least 50 mol%.

**18.** Polymer beads according to any of claims 11 to 17, which consist to an extent of at least 0.5% by weight of copolymerized crosslinker b).

**19.** The use of the polymer beads according to any of claims 11 to 18 for producing hygiene articles.

**20.** A hygiene article comprising polymer beads according to any of claims 11 to 18.

**Revendications**

**1.** Procédé pour la production de particules de polymère absorbant l'eau, par polymérisation de gouttes d'une solution de monomères, contenant

a) au moins un monomère à insaturation éthylénique,
b) au moins un agent de réticulation,
c) au moins un amorceur,
d) de l'eau,

dans une phase gazeuse entourant les gouttes, la polymérisation s'effectuant en phase homogène dans la goutte, **caractérisé en ce que** la solution de monomères contient au moins 0,5 % en poids de l'agent de réticulation b) par rapport au monomère a) et les particules de polymère ont un diamètre moyen d'au moins 150 μm.

**2.** Procédé selon la revendication 1, **caractérisé en ce que** le monomère a) comporte au moins un groupe acide.

**3.** Procédé selon la revendication 2, **caractérisé en ce que** les groupes acides du monomère a) sont au moins partiellement neutralisés.

**4.** Procédé selon l'une quelconque des revendications 1 à 3, **caractérisé en ce que** le monomère a) consiste à raison d'au moins 50 % en moles en acide acrylique.

**5.** Procédé selon l'une quelconque des revendications 1 à 4, **caractérisé en ce que** les particules de polymère ont un diamètre moyen d'au moins 200 μm.

**6.** Procédé selon l'une quelconque des revendications 1 à 5, **caractérisé en ce qu'**au moins 90 % en poids des particules de polymère ont un diamètre de 100 à 800 μm.

**7.** Procédé selon l'une quelconque des revendications 1 à 6, **caractérisé en ce qu'**un gaz vecteur traverse la chambre de réaction.

**8.** Procédé selon la revendication 7, **caractérisé en ce que** le gaz vecteur quittant la chambre de réaction est au moins partiellement recyclé après un passage.

**9.** Procédé selon l'une quelconque des revendications 1 à 8, **caractérisé en ce que** la teneur en oxygène du gaz vecteur vaut de 0,001 à 0,15 % en volume.

**10.** Procédé selon l'une quelconque des revendications 1 à 9, **caractérisé en ce que** les particules de polymère obtenues, sont séchées et/ou post-réticulées dans au moins une autre étape du procédé.

**11.** Particules de polymère absorbant l'eau, les particules de polymère présentant une sphéricité d'au moins 0,84, une teneur en solvant hydrophobe de moins de 0,005 % en poids et une perméabilité d'au moins 5 x $10^{-7}$ $cm^3s/g$.

**12.** Particules de polymère selon la revendication 11, **caractérisées en ce que** les particules de polymère présentent une capacité de rétention centrifuge d'au moins 10 g/g.

**13.** Particules de polymère selon la revendication 11 ou 12, **caractérisées en ce que** les particules de polymère ont un diamètre moyen d'au moins 200 $\mu$m.

**14.** Particules de polymère selon l'une quelconque des revendications 11 à 13, **caractérisées en ce qu'**au moins 90 % en poids des particules de polymère ont un diamètre de 100 à 800 $\mu$m.

**15.** Particules de polymère selon l'une quelconque des revendications 11 à 14, **caractérisées en ce que** les particules de polymère sont au moins en partie constituées de monomères a) polymérisés portant des groupes acides.

**16.** Particules de polymère selon la revendication 15, **caractérisées en ce que** les groupes acides des monomères a) polymérisés sont au moins partiellement neutralisés.

**17.** Particules de polymère selon la revendication 15 ou 16, **caractérisées en ce** le monomère a) consiste à raison d'au moins 50 % en moles en acide acrylique.

**18.** Particules de polymère selon l'une quelconque des revendications 11 à 17, **caractérisées en ce que** les particules de polymère sont constituées à raison d'au moins 0,5 % en poids d'agent de réticulation b) incorporé par polymérisation.

**19.** Utilisation des particules de polymère selon l'une quelconque des revendications 11 à 18, pour la fabrication d'articles d'hygiène.

**20.** Article d'hygiène, contenant des particules de polymère selon l'une quelconque des revendications 11 à 18.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 0348180 A
- WO 9640427 A
- US 5269980 A
- DE 10314466 A
- DE 10340253 A
- DE 102004024437 A
- DE 102005002412
- DE 102006001596
- DE 102004042946 A
- DE 102004042948 A
- DE 102004042955 A
- DE 102005019398
- EP 0530438 A
- EP 0547847 A
- EP 0559476 A
- EP 0632068 A
- WO 9321237 A
- WO 03104299 A
- WO 03104300 A
- WO 03104301 A
- DE 10331450 A
- DE 10331456 A
- DE 10355401 A
- DE 19543368 A
- DE 19646484 A
- WO 9015830 A
- WO 0232962 A
- EP 0343427 A
- DE 4308842 A
- US 6338438 B
- EP 0083022 A
- EP 0543303 A
- EP 0937736 A
- DE 3314019 C
- DE 3523617 C
- EP 0450922 A
- DE 10204938 A
- US 6239230 B
- DE 4020780 A
- DE 198075022 A
- DE 19807992 A
- DE 198545732 A
- DE 19854574 A
- DE 10204937 A
- DE 10334584 A
- EP 1199327 A
- WO 03031482 A
- WO 9957355 A
- EP 1023883 A
- WO 9526209 A
- DE 19604601 A
- EP 0316518 A
- EP 0202127 A
- WO 0065084 A
- WO 0065348 A
- WO 0035502 A
- DE 19737434 A
- WO 9808439 A
- WO 9524173 A
- WO 9111977 A
- EP 0389023 A
- WO 9425077 A
- WO 9701317 A
- WO 9918905 A
- EP 0834297 A
- US 5762644 A
- US 5895381 A
- WO 9857609 A
- WO 0065083 A
- WO 0069485 A
- WO 0069484 A
- WO 0069481 A
- US 6123693 A
- EP 1104666 A
- WO 0124755 A
- WO 0100115 A
- EP 0105373 A
- WO 0141692 A
- EP 1074233 A
- WO 9848753 A
- WO 9841179 A
- WO 9709022 A
- WO 9846182 A
- WO 9846181 A
- WO 0143679 A
- WO 0143680 A
- WO 0061052 A
- EP 1108408 A
- WO 0133962 A
- DE 10020662 A
- WO 0101910 A
- WO 0101908 A
- WO 0101909 A
- WO 0101906 A
- WO 0101905 A
- WO 0124729 A
- EP 0311344 A
- EP 0850623 A

- WO 9526207 A
- EP 0894502 A
- EP 0850616 A
- WO 9822063 A
- WO 9749365 A
- EP 0903134 A
- EP 0887060 A
- EP 0887059 A
- EP 0887058 A
- EP 0887057 A
- EP 0887056 A
- EP 0931530 A
- WO 9925284 A
- WO 9322998 A
- WO 9820916 A
- EP 0306262 A
- WO 9945973 A
- WO 9828478 A
- EP 0615736 A
- WO 0036216 A
- US 3556932 A
- WO 9111162 A
- US 3224926 A
- US 3440135 A
- US 3932209 A
- US 4035147 A
- US 4822453 A
- US 4888093 A
- US 4898642 A
- US 5137537 A
- EP 0640330 A

**In der Beschreibung aufgeführte Nicht-Patentliteratur**

- **F.L. Buchholz ; A.T. Graham.** Modern Superabsorbent Polymer Technology. Wiley-VCH, 1998, 71-103
- **von Robert F. Gould.** Kontaktwinkel, Benetzbarkeit und Adhäsion. American Chemical Society, 1964